# EUROPEAN PATENT APPLICATION

(11) **EP 2 067 470 A1**
(43) Date of publication of application: **10.06.2009**
(21) Application number: 07122085.9
(22) Date of filing: 03.12.2007
(51) Int. Cl.: A61K 9/16, A61K 9/20, A61K 9/28, A61K 31/41, A61K 31/54

(54) **Pharmaceutical compositions containing valsartan and process for its preparation**

(71) Applicant: Laboratorios Lesvi, S.L., 08970 Sant Joan Despi (Barcelona) (ES)
(72) Inventor: Martín Gallart, Gema, 08950, Esplugues de Llobregat (Barcelona (ES); Úbeda Pèrez, Carmen, 08348, Cabrils (Barcelona) (ES); Díez Martín, Ignacio, 08980, Sant Feliu de Llobregat (Barcelona) (ES)
(74) Representative: Barlocci, Anna

(57) **Abstract**

Oral pharmaceutical composition comprising (a) a plurality of granules consisting of valsartan, or a pharmaceutically acceptable salt thereof, or a solvate, including hydrates, or a solid form thereof, as active ingredient in an amount comprised between 60 and 98% to the total weight of the granule, at least one disintegrant in an amount comprised between 2 and 5% to the total weight of the granule, a binder in an amount comprised between 0 and 10% to the total weight of the granule and, optionally, a diuretic in an amount comprised between 0 and 25%; and (b) a pharmaceutically acceptable alkaline-earth metal carbonate as diluent, together with appropriate amounts of other pharmaceutical excipients or carriers and optionally a diuretic. The diuretic of the pharmaceutical formulation is hydrochlorothiazide.

## Description

### FIELD OF THE INVENTION

The present invention relates to new pharmaceutical compositions for the oral administration of Valsartan, or a pharmaceutically acceptable salt or a polymorph thereof, optionally combined with a diuretic, and to its preparation process.

### BACKGROUND OF THE INVENTION

Valsartan is a pharmaceutical active compound useful for the treatment of hypertension and heart failure. The chemical name of Valsartan is N-(1-oxopentyl)-N-[[2'-(1H-tetrazol-5-yl)[1,1'-biphenyl]-4-yl]methyl]-L-Valine with CAS Registry number 137862-53-4 and structural formula I.

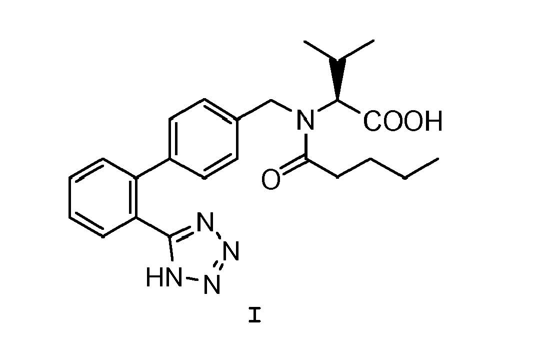

The synthesis of Valsartan is disclosed in EP0443983, EP1747190 and WO2007045675 among other patent and patent applications.

Valsartan is marketed as Diovan® as a free acid in 40, 80 and 160 mg alone and as Co-Diovan® in combination with hydrochlorothiazide (HCTZ) in 80/12.5 mg and 160/25 mg strengths.

Different formulations of Valsartan are disclosed in EP0443983 (using wet granulation of the Valsartan and a part of the diluents), EP0883401 (hard gelatine capsule), EP0914119 (compressed solid oral dosage containing more than 35% Valsartan), and EP1588706 (containing more than 30% microcrystalline cellulose and a ratio of Valsartan and microcrystalline cellulose of 2:1 to 0.3:1).

As stated in EP0914119B1 Valsartan is difficult to formulate and has a low density, thus different formulations are needed to address a good bioavailability and stability that could be tabletted in an easy manner. To obtain a good bioavailability, it is desired to obtain disgregation times for the formulations of Valsartan lower than 3 minutes.

For all these reasons, there is still a need for developing pharmaceutical compositions which incorporate Valsartan and methods for preparing said compositions with an improved physical stability which allows proper disgregation and bioavailability properties.

### SUMMARY OF THE INVENTION

The inventors have found that a pharmaceutical composition having a plurality of granules containing valsartan and at least one disintegrant in an appropriate amount; and also having an alkaline earth metal carbonate as diluent, has an appropriate flowability to be properly tabletted, and also shows a good stabiltiy and short disgregation times.

Thus, according to an aspect of the present invention, it is provided an oral pharmaceutical composition comprising: (a) a plurality of granules, said granules consisting of valsartan, or a pharmaceutically acceptable salt, or a solvate, including hydrates, or a solid form thereof, as active ingredient in an amount comprised between 60 and 98% to the total weight of the granule, at least one disintegrant in an amount comprised between 2 and 5% to the total weight of the granule, a binder in an amount comprised between 0 and 10% to the total weight of the granule, and a diuretic in an amount comprised between 0 and 25%; and (b) an alkaline earth metal carbonate as diluent, together with appropriate amounts of other pharmaceutical excipients or carriers and, optionally, a diuretic.

According to a second aspect of the present invention it is provided a process for preparing the pharmaceutical composition as defined above comprising:
(a) Preparing granules of Valsartan and a disintegrant;
(b) Mixing the granules with the pharmaceutically acceptable alkaline-earth metal carbonate, the remaining pharmaceutically acceptable excipients, including the remaining disintegrant;
(c) Mixing the previous mixture with a lubricant; and
(d) Compress the final mixture to form a tablet.

A third aspect of the present invention is a pharmaceutical composition of Valsartan further characterized by the fact that it also includes a diuretic. The preferred diuretic is hydrochlorothiazide.

### DETAILED DESCRIPTION OF THE INVENTION

As it is mentioned above, the pharmaceutical composition containing the granules of the present invention and an alkaline earth metal carbonate as diluent is advantageous because is easy to tablet because it has an appropriate flowability, is time stable and shows short disgregation times. It has been found that the Valsartan bulk material has an apparent density of 0.27-0.39 g/cm³, and that preparing said granules of Valsartan with a disintegrant, and optionally a binder, the density of the granules rises to higher than 0.4 g/cm³, but usually not higher than 0.75 g/cm³. It has surprisingly found that this small increase in the density is sufficient to greatly improve the flowability properties of the final mixture.

When the Valsartan was granulated including the diluents, the glidants and the lubricants, it was found that the flowability was not acceptable, but when the Valsartan was granulated only with a disintegrant and, optionally, with a binder, the flowability was acceptable during all the tabletting process.

The flowability has been measured according to the European Pharmacopoeia 5.0 (Ph. Eur. 5.0) item 2.9.16-2, and is represented in seconds (s), the higher the time the slower the mixture flows. The desired time is less than 6 seconds.

The tapped density is the density of the bulk product or mixture. The compacted density has been measured according to the Spanish Pharmacopoeia of the year 1997 item 2.9.15.

The stability has been measured in an Open Dish study and in packaged product using aluminium/aluminium or aluminium/PVC blister,

The first aspect of the present invention is to provide an oral pharmaceutical composition comprising: (a) a plurality of granules, said granules consisting of valsartan, or a pharmaceutically acceptable salt, or a solvate, including hydrates, or a solid form thereof, as active ingredient in an amount comprised between 60 and 98% to the total weight of the granule, at least one disintegrant in an amount comprised between 2 and 5% to the total weight of the granule, a binder in an amount comprised between 0 and 10% to the total weight of the granule, and a diuretic in an amount comprised between 0 and 25%; and (b) an alkaline earth metal carbonate as diluent, together with appropriate amounts of other pharmaceutical excipients or carriers and, optionally, a diuretic.

In the present description, when the term Valsartan is used it is meant to include any of its pharmaceutically acceptable salts, its solvates, including hydrates, or its solid forms (amorphous or polymorphic forms). The preferred form is the Valsartan essentially amorphous obtained as described in EP1511739.

The granules of the pharmaceutical composition are characterized by the fact that the disintegrant is present in an amount comprised between 2,2-3,5% relative to the total weight of the granule and the most preferred amount of disintegrant is 2,5-3,2% relative to the total weight of the granule.

Preferably, the disintegrant is selected from the group consisting of: crospovidone, carboxymethyl cellulose sodium, low substituted hydroxypropyl cellulose, and carboxymethyl starch sodium.

Optionally, the granules further comprise a binder. The preferred amount of binder, if present, is 2-9% relative to the total weight of the granule, the most preferred is 3-8%.

Preferably, the binder is selected from the group consisting of: povidone, hydroxypropyl methylcellulose, pregelatinized starch, low substituted hydroxypropyl cellulose, and starch.

The pharmaceutical composition of the present invention is also characterized by the presence of a pharmaceutically acceptable alkaline-earth metal carbonate. In a preferred embodiment, the pharmaceutically acceptable alkaline-earth metal is calcium carbonate. In another preferred embodiment is magnesium carbonate. The last one is the most preferred.

Other appropriate pharmaceutical excipients that can be present in the pharmaceutical composition are selected from the group consisting of: one or more diluents, one or more glidants, and one or more lubricants.

Preferably, the diluents are selected from microcrystalline cellulose, cellulose powder, anhydrous lactose, calcium hydrogen phosphate dihydrate, mannitol, and starch. In a preferred embodiment of the pharmaceutical composition of the present invention, the diluents, including the pharmaceutically acceptable alkaline-earth metal carbonate, are present in a 50-70% of the total weight. In a more preferred embodiment, the percentage of diluents is 53-60% of the total weight. Preferably, the pharmaceutically acceptable alkaline-earth metal carbonate represents a 30-70% in weight of the diluents, more preferably represents the 40-60% in weight of the diluents, and even more preferably represents the 45-55% in weight of the diluents.

Preferably, the glidants are selected from anhydrous colloidal silica, magnesium trisilicate, and talc.

Also preferably, the lubricants are selected from magnesium stearate, stearic acid, glycerol dibehenate, and talc.

The pharmaceutical composition of the present invention can also include a diuretic, preferably hydrochlorothiazide. Said compound can be present in the Valsartan granules, it can be out of the Valsartan granules or can be in and out of the granules.

In a particular embodiment, the pharmaceutical composition of the present invention is a tablet, and optionally this tablet is film coated.

The coating can be chosen from the from the coatings available to the skilled in the art without special requirements, such as hydroxypropylmethyl cellulose, polyvinyl or alcohol.

Preferably, the pharmaceutical composition of the present invention has the following preferred quantitative composition:

| | % |
|---|---|
| Valsartan | 25-40 |
| Diluents | 50-65 |
| Binders | 0-5 |
| Disintegrants | 5-10 |
| Glidants | 0,1-0,4 |
| Lubricant | 0,5-1,5 |
| Diuretic | 0-20 |

wherein the pharmaceutically acceptable alkaline-earth metal carbonate, preferably magnesium carbonate, represents a 30-70% of the diluents, preferably represents the 40-60% of the diluents, more preferably represents the 45-55% of the diluents, and the diluents, binders, disintegrants, glidants and lubricants are the same as defined earlier.

The pharmaceutical composition of the present invention can be prepared by a process comprising the following steps:
a) Preparing granules of Valsartan and a disintegrant;
b) Mixing the granules with pharmaceutically acceptable alkaline-earth metal carbonate, the remaining pharmaceutically acceptable excipients, including the remaining disintegrant;
c) Mixing the previous mixture with the lubricant; and
d) Compress the mixture to form a tablet.

Additionally, when the pharmaceutical composition contains a diuretic, said diuretic can be added in the step a) and/or step b) of the above process.

The granules of Valsartan of step a), optionally containing a diuretic, can be prepared by different procedures:
Procedure 1; Compactation: compacting a mixture of Valsartan, a disintegrant, and optionally a diuretic.
Procedure 2a; Wet granulation:
   a) Mixing the Valsartan, the disintegrant and, optionally, a diuretic.
   b) Adding an aqueous or alcoholic solution of a binder.
   c) Granulate, dry and sieve the granules.
Procedure 2b; wet granulation:
   a) Mixing the Valsartan, the disintegrant, the binder and, optionally, a diuretic.
   b) Adding water, alcohol or their mixtures.
   c) Granulate, dry and sieve the granules.

Preferably, the alcohol used in the previous process is selected from ethanol and 2-propanol.

When the granules are prepared by a wet granulation process, the amount of disintegrant of step a) is present in a 2-5% relative to the total weight of the granule, the preferred amount of disintegrant is 2,2-3,5% relative to the total weight of the granule and the most preferred amount of disintegrant is 2,5-3,2% relative to the total weight of the granule.

In another preferred embodiment, the process includes an additional step where the tablet is further coated.

Using this formulation and this process it is obtained a pharmaceutical composition containing Valsartan with proper pharmaceutical properties.

In the following, the present invention is further illustrated by examples. They should in no case be interpreted as a limitation of the scope of the invention as defined in the claims.

### EXAMPLES

### Comparative Example 1: wet granulation

| **Quantitative composition:** | **(g)** | **%** |
|---|---|---|
| Valsartan | 80,000 | 34,78 |
| Microcrystalline Cellulose PH-102 | 142,525 | 61,97 |
| Crospovidone | 4,600 | 2,00 |
| Anhydrous colloidal silica | 0,575 | 0,25 |
| Magnesium stearate | 2,300 | 1,00 |
| Purified water * | 400,0 | |
| Total weight | 230,0 | |

| | | |
|---|---|---|
| * solvent which disappears during the manufacturing process. | | |

### Detailed description of the manufacturing process:

Valsartan and the microcrystalline cellulose are mixed and granulated in a high shear mixer with purified water, dried and sieved. Then, the crospovidone and the anhydrous colloidal silica are added and mixed with the granules, finally is added the magnesium stearate and all the mixture is mixed. Although the flowability is not acceptable for the tabletting process (10 s), the resulting mixture is tabletted, but the tablets were not homogeneous. The formulation suffers from lack homogeneity.

### Comparative Example 2: direct compression

| **Quantitative composition:** | **(g)** | **%** |
|---|---|---|
| Valsartan | 80,000 | 34,78 |
| Microcrystalline Cellulose PH-102 | 135,625 | 58,97 |
| Crospovidone | 11,500 | 5,00 |
| Anhydrous colloidal silica | 0,575 | 0,25 |
| Magnesium stearate | 2,300 | 1,00 |
| Total weight | 230,0 | |

### Detailed description of the manufacturing process:

Valsartan is mixed with all the excipients except the magnesium stearate. Then the magnesium stearate is added and the mixture is mixed. Although the flowability is not acceptable for the tabletting process (9 s), the resulting mixture is tabletted by direct compression and resulted in non homogeneous tablets. Even though Open Dish studies were performed on the resulting tablets in bulk and in closed amber glass flasks at 40°C at 75% RH and 15 days, the resulting disgregation times are higher than 30 minutes. This formulation has also low homogeneity.

### Comparative Example 3: Direct compression film coated

| **Quantitative composition:** | **(g)** | **%** |
|---|---|---|
| Valsartan | 80,000 | 33,90 |
| Microcrystalline Cellulose PH-102 | 67,813 | 28,73 |
| Magnesium carbonate | 67,813 | 28,73 |
| Crospovidone | 11,500 | 4,87 |
| Anhydrous colloidal silica | 0,575 | 0,24 |
| Magnesium stearate | 2,300 | 0,97 |
| Opadry 03B34439 Pink | 6,000 | 2,54 |
| Purified water* | 54,0 | |
| Total weight | 236,0 | |

| | | |
|---|---|---|
| * solvent which disappears during the film coating process. | | |

### Detailed description of the manufacturing process:

Valsartan is mixed with the microcrystalline cellulose, the magnesium carbonate, the crospovidone, and the anhydrous colloidal silica. Then the magnesium stearate is added and mixed. Although the flowability is not acceptable (10 s) for the tabletting process, the resulting mixture is tabletted by direct compression. Finally the tablets are film coated with Opadry 03B34439 Pink. Stability studies in Open Dish and packaged tablets in closed amber glass flasks at 40°C at 75% RH and 15 days were performed, the resulting disgregation times are higher than 15 minutes.

### Example 1. wet granulation

| **Quantitative composition:** | **(g)** | **%** |
|---|---|---|
| Valsartan | 80,000 | 34,78 |
| Microcrystalline Cellulose PH-102 | 64,613 | 28,09 |
| Magnesium carbonate | 64,613 | 28,09 |
| Povidone K-25 | 4,000 | 1,74 |
| Crospovidone | 13,900 | 6,04 |
| Anhydrous colloidal silica | 0,575 | 0,25 |
| Magnesium stearate | 2,300 | 1,00 |
| purified water* | 48 | |
| Total weight | 230,0 | |

| | | |
|---|---|---|
| * solvent which disappears during the manufacturing process. | | |

### Detailed description of the manufacturing process:

Valsartan and 3% crospovidone (relative to the total amount of Valsartan) are granulated in a high shear mixer with an aqueous solution of the povidone, and then dried and sieved. The resulting granules are mixed with the remaining crospovidone and the other excipients (microcrystalline cellulose, magnesium carbonate and the anhydrous colloidal silica). Finally, the magnesium stearate is added and mixed with the previous granules. The flowability is acceptable for the tabletting process (6 s) and the resulting mixture is tabletted.

### Example 2: wet granulation

| **Quantitative composition:** | **(g)** | **%** |
|---|---|---|
| Valsartan | 80,000 | 34,78 |
| Microcrystalline Cellulose PH-102 | 64,613 | 28,09 |
| Magnesium carbonate | 64,613 | 28,09 |
| Povidone K-25 | 4,000 | 1,74 |
| Crospovidone | 13,900 | 6,04 |
| Anhydrous colloidal silica | 0,575 | 0,25 |
| Magnesium stearate | 2,300 | 1,00 |
| alcohol* | 24 | |
| Total weight | 230,0 | |

| | | |
|---|---|---|
| * solvent which disappears during the manufacturing process. | | |

### Detailed description of the manufacturing process:

This composition is prepared following the procedure of example 1 using alcohol instead of water. The flowability is acceptable for the tabletting process (5 s) and the resulting mixture is tabletted. Thus, the tablet is homogeneous.

### Example 3: wet granulation

| **Quantitative composition:** | **(g)** | **%** |
|---|---|---|
| Valsartan | 80,000 | 34,78 |
| Microcrystalline Cellulose PH-102 | 63,213 | 27,48 |
| Magnesium carbonate | 63,213 | 27,48 |
| Povidone K-25 | 6,800 | 2,96 |
| Crospovidone | 13,900 | 6,04 |
| Anhydrous colloidal silica | 0,575 | 0,25 |
| Magnesium stearate | 2,300 | 1,00 |
| purified water* | 26 | |
| Total weight | 230,0 | |

| | | |
|---|---|---|
| * solvent which disappears during the manufacturing process. | | |

### Detailed description of the manufacturing process:

This composition is prepared following the procedure of example 1. The density of the granules is 0.413 g/cm³. The flowability is acceptable (6 s) for the tabletting process and the resulting mixture is tabletted. Stability studies in Open Dish and in package tablets in aluminium/aluminium and aluminium/PVC blisters at 40°C at 75% RH during 6 months were performed, the resulting disgregation times are lower than 2 minutes.

### Example 4: wet granulation

| **Quantitative composition:** | **(g)** | **%** |
|---|---|---|
| Valsartan | 80,000 | 34,78 |
| Microcrystalline Cellulose PH-102 | 65,143 | 28,32 |
| Magnesium carbonate | 65,143 | 28,32 |
| Povidone K-25 | 2,980 | 1,30 |
| Crospovidone | 13,900 | 6,04 |
| Anhydrous colloidal silica | 0,575 | 0,25 |
| Magnesium stearate | 2,300 | 1,00 |
| purified water* | 29 | |
| Total weight | 230,0 | |

| | | |
|---|---|---|
| * solvent which disappears during the wet granulation process. | | |

### Detailed description of the manufacturing process:

This composition is prepared following the procedure of example 1. The density of the granules is 0.455 g/cm³. The flowability and the compressibility are acceptable for the tabletting process (4 s) and the resulting mixture is tabletted. Stability studies on the resulting tablets in Open Dish and in packaged tablets in aluminium/aluminium and aluminium/PVC blisters at 40°C at 75% RH during 6 months have been performed; the resulting disgregation times are lower than 1 minute.

### Example 5: Direct compression

| **Quantitative composition:** | **(g)** | **%** |
|---|---|---|
| Valsartan | 80,000 | 33,90 |
| Microcrystalline Cellulose PH-102 | 66,613 | 28,23 |
| Magnesium carbonate | 66,613 | 28,23 |
| Crospovidone | 13,900 | 5,89 |
| Anhydrous colloidal silica | 0,575 | 0,24 |
| Magnesium stearate | 2,300 | 0,97 |
| Opadry 03B34439 Pink | 6,000 | 2,54 |
| Purified water^{*} | 54,0 | |
| Total weight | 236,0 | |

| | | |
|---|---|---|
| * solvent which disappears during the film coating process. | | |

### Detailed description of the manufacturing process:

Valsartan and 3% crospovidone (relative to the total amount of Valsartan) are compacted at 11 KN/cm². The resulting mixture is mixed with the remaining crospovidone and the other excipients (microcrystalline cellulose, magnesium carbonate, the anhydrous colloidal silica and the magnesium stearate). The flowability and the compressibility are acceptable (6 s) for the tabletting process and the resulting mixture is tabletted Stability studies on the resulting tablets in Open Dish and in packaged tablets in aluminium/aluminium and aluminium/PVC blisters at 40°C at 75% RH during 6 months have been performed; the resulting disgregation times are lower than 2 minutes.

### Example 6: wet granulation

| **Quantitative composition:** | **(g)** | **%** |
|---|---|---|
| Valsartan | 80,00 | 34,78 |
| Microcrystalline Cellulose PH-102 | 31,74 | 13,80 |
| Cellulose Powder | 33,38 | 14,51 |
| Magnesium carbonate | 65,11 | 28,31 |
| Povidone K-25 | 3,00 | 1,30 |
| Crospovidone | 13,90 | 6,04 |
| Anhydrous colloidal silica | 0,58 | 0,25 |
| Magnesium stearate | 2,30 | 1,00 |
| purified water* | 29 | |
| Total weight | 230,00 | |

| | | |
|---|---|---|
| * solvent which disappears during the wet granulation process. | | |

### Detailed description of the manufacturing process:

This composition is prepared following the procedure of example 1. The density of the granules is 0.446 g/cm³. The flowability and the compressibility are acceptable for the tabletting process (6 s) and the resulting mixture is tabletted. Stability studies on the resulting tablets in Open Dish and in packaged tablets in aluminium/aluminium and aluminium/PVC blisters at 40°C at 75% RH during 30 days have been performed; the resulting disgregation times are lower than 3 minutes.

### Example 7: wet granulation, hydrochlorothiazide combination

| **Quantitative composition:** | **(g)** | **%** |
|---|---|---|
| Valsartan | 80,000 | 32,98 |
| Microcrystalline Cellulose PH-102 | 32,571 | 13,43 |
| Cellulose powder | 32,571 | 13,43 |
| Magnesium carbonate | 65,143 | 26,86 |
| Hydrochlorothiazide | 12,500 | 5,15 |
| Povidone K-25 | 2,980 | 1,23 |
| Crospovidone | 13,900 | 5,73 |
| Anhydrous colloidal silica | 0,575 | 0,24 |
| Magnesium stearate | 2,300 | 0,95 |
| purified water* | 29 | |
| Total weight | 242,5 | |

| | | |
|---|---|---|
| * solvent which disappears during the wet granulation process. | | |

Detailed description of the manufacturing process:

This composition is prepared following the procedure of example 1 mixing the hydrochlorothiazide with the magnesium carbonate and the remaining excipients. The flowability and the compressibility are acceptable for the tabletting process (5 s) and the resulting mixture is tabletted.

## Claims

1. An oral pharmaceutical composition comprising:
(a) a plurality of granules, said granules consisting of valsartan, or a pharmaceutically acceptable salt thereof, or a solvate, including hydrates, or a solid form thereof, as active ingredient in an amount comprised between 60 and 98% to the total weight of the granule, at least one disintegrant in an amount comprised between 2 and 5% to the total weight of the granule, a binder in an amount comprised between 0 and 10% to the total weight of the granule, and a diuretic in an amount comprised between 0 and 25% to the total weight of the granule; and
(b) a pharmaceutically acceptable alkaline-earth metal carbonate as diluent, together with appropriate amounts of other pharmaceutical excipients or carriers and, optionally, a diuretic.

2. The pharmaceutical composition according to claim 1, wherein the granules have a density between 0.4-0.75 g/cm³.

3. The pharmaceutical composition according to any of the claims 1-2, wherein the amount of disintegrant is comprised between 2.2 and 3.5% to the total weight of the granule.

4. The pharmaceutical composition according to claim 3, wherein the amount of disintegrant is comprised between 2.5 and 3.2% to the total weight of the granule.

5. The pharmaceutical composition according to any of the claims 1-4, wherein the disintegrant is selected from the group consisting: crospovidone, carboxymethyl cellulose sodium, low substituted hydroxypropyl cellulose, and carboxymethyl starch sodium.

6. The pharmaceutical composition according to any of the claims 1-5, wherein the binder is present in an amount comprised between 2-9% relative to the total weight of the granule.

7. The pharmaceutical composition according to claim 6, the amount of binder is comprised between 3-8% relative to the total weight of the granule.

8. The pharmaceutical composition according to any of the claims 6-7, wherein the binder is selected from the group consisting of povidone, hydroxypropyl methylcellulose, pregelatinized starch, low substituted hydroxypropyl cellulose, and starch.

9. The pharmaceutical composition according to any of the claims 1-8, wherein the pharmaceutically acceptable alkaline-earth metal carbonate is magnesium carbonate.

10. The pharmaceutical composition according to any of the claims 1-9, wherein the diluents, including the magnesium carbonate, are present in a 50-70% of the total weight of the formulation.

11. The pharmaceutical composition according to claim 10, wherein the alkaline earth metal carbonate is present in an amount comprised between 44-55% in weight of the diluents.

12. The pharmaceutical composition according to any of the claims 1-11, which is a tablet.

13. The pharmaceutical composition according to any of the claims 1-12, further comprising hydrochlorothiazide as a diuretic.

14. The pharmaceutical composition according to claim 13, wherein the hydrochlorothiazide is out of the Valsartan granules.

15. The pharmaceutical composition according to any of the claims 1-14 which has the following quantitative composition:
| | % |
|---|---|
| Valsartan | 25-40 |
| Diluents | 50-65 |
| Binders | 0-5 |
| Disintegrants | 5-10 |
| Glidants | 0,1-0,4 |
| Lubricant | 0,5-1,5 |
| Diuretic | 0-20 |
wherein the pharmaceutically acceptable alkaline-earth metal carbonate represents a 45-55% of the diluent content.

16. A process for preparing a pharmaceutical composition according to any of the preceding claims comprising:
(a) Preparing granules of Valsartan, a disintegrant;
(b) Mixing the granules with the pharmaceutically acceptable alkaline-earth metal carbonate, the remaining pharmaceutically acceptable excipients, including the remaining disintegrant;
(c) Mixing the previous mixture with the lubricant; and
(d) Compress the final mixture to form a tablet.

17. The process of claim 16 wherein a diuretic is added to step a) and/or step b).

18. The process according to claim 16, wherein the granules are prepared by compacting a mixture of Valsartan and a disintegrant.

19. The process according to claim 16, wherein the granules are prepared by:
(a) Mixing the Valsartan, the disintegrant and, optionally, a diuretic.
(b) Adding an aqueous or alcoholic solution of a binder.
(c) Granulate, dry and sieve the granules.

20. The process according to claim 16, wherein the granules are prepared by:
(a) Mixing the Valsartan, the disintegrant, the binder and, optionally, a diuretic.
(b) Adding water, alcohol or their mixtures.
(c) Granulate, dry and sieve the granules.

21. The process according to any of the claims 16-20, wherein the alcohol is selected from ethanol and 2-propanol.
